# FASCICULE DE BREVET EUROPEEN

(11) **EP 1 258 236 B1**
(45) Date de publication et mention de la délivrance du brevet: **11.11.2009**
(21) Numéro de dépôt: 02291125.9
(22) Date de dépôt: 03.05.2002
(51) Int. Cl.: A61Q 5/12, A61Q 5/02

(54) **Composition de conditionnement des cheveux comprenant un mélange d'alcools gras**
Haarkonditionierungsmittel enthaltend eine Mischung von Fettalkoholen
Hair conditioning composition containing a mixture of fatty alcohols

(30) Priorité: 15.05.2001 FR 0106383
(43) Date de publication de la demande: 20.11.2002
(73) Titulaire: L'ORÉAL, 75008 Paris (FR)
(72) Inventeur: Müller, Rainer, 76344 Leopoldshafen (DE)
(74) Mandataire: Casalonga, Axel

(56) Documents cités:
- EP-A- 0 976 393
- EP-A- 1 093 808
- WO-A-01/00149
- WO-A-99/62492
- DE-A- 19 851 451
- FR-A- 2 745 016

## Description

La présente invention est relative à une composition de conditionnement des cheveux comprenant un mélange particulier d'alcools gras et à un procédé de traitement cosmétique des cheveux.

Les après-shampoings sont des compositions de conditionnement bien connues dans la technique. Ce sont en général des 6mulsions d'alcools gras.

Les alcools gras ainsi utilisés sont bien connus de l'homme de métier comme étant notamment des agents conditionneurs et/ou des agents épaississants.

En effet, des alcools primaires à longue chaîne saturée comportant en moyenne de 25 à 45 atomes de carbone sont décrits dans les documents US 5 213 716 et US 5 348 736 de Colgate.

Les documents WO 99/62467 et WO 99/62492 de Procter et Gamble décrivent aussi des compositions de conditionnement des cheveux comprenant entre autres, un mélange d'alcools cétylique, stéarylique et béhénylique, l'alcool béhénylique constituant jusqu'à 15 % du mélange d'alcools.

EP 1 093 808 A de la société L'OREAL décrit des compositions cosmétiques comprenant, dans un milieu cosmétiquement acceptable, l'association d'au moins un copolymère siliconé et d'au moins un polymère cationique pour limiter, voire supprimer l'alourdissement, le manque de lissage et de douceur des cheveux, obtenus avec des compositions à base de polymères cationiques.

WO 01/00149 A de la société L'OREAL décrit une composition cosmétique nacrante comprenant, dans un milieu cosmétiquement acceptable :
- au moins une base tensioactive constituée de tensioactifs choisis parmi les tensioactifs anioniques, non ioniques, amphotères, cationiques et leurs mélanges,
- au moins un alcool gras linéaire et saturé à longue chaîne en C₂₂,
- au moins un alcool gras linéaire et saturé à longue chaîne en C₁₈, et
- au moins un agent opacifiant et/ou nacrant,
le rapport alcool gras en C₁₈/alcool gras en C₂₂ étant supérieur à 0,15.

EP 0 976 393 A de la société L'OREAL, décrit une composition cosmétique nacrante comprenant, dans un milieu cosmétiquement acceptable :
- au moins une base tensioactive constituée de tensioactifs choisis parmi les tensioactifs anioniques, non ioniques, amphotères, cationiques et leurs mélanges,
- au moins un alcool gras ou un mélange d'alcools gras linéaires et saturés comportant au moins 50 % en poids d'un alcool en C₂₂, et
- au moins un agent opacifiant et/ou nacrant.

WO 99/62492 A de la société The Procter & Gamble décrit une composition présentant des propriétés de conditionnement des cheveux améliorées comprenant :
(1) au moins 3 % d'un composé de température de fusion élevée,
(2) un agent émulsifiant choisi parmi les amines, les bétaïnes, les composés non ioniques et leurs mélanges,
(3) un composé quaternaire, et
(4) un véhicule aqueux.

Après application des après-shampoings de l'art antérieur, en particulier sur des cheveux fins, la demanderesse a constaté que les cheveux étaient chargés et manquaient de légèreté, notamment après des usages répétés.

Elle a donc cherché à obtenir un dépôt moins important d'alcools gras et d'agents conditionneurs sur les cheveux fins tout en conservant de bonnets propriétés de conditionnement telles que la souplesse et le caractère lisse des cheveux humides, et une malléabilité des cheveux séchés.

La demanderesse a découvert de manière surprenante qu'en limitant la longueur de la chaîne carbonée des alcools gras dans les apres-shampoings. à un nombre d'atomes de carbone compris entre 20 et 24 atomes de carbone, on pouvait diminuer le dépôt d'alcool gras sur les cheveux ainsi que le dépôt des agents conditionneurs qui co-précipitent avec l'alcool gras, comme par exemple, les silicones et les tensioactifs cationiques. Ces compositions permettent alors de surmonter les inconvénients décrits ci-dessus, et d'obtenir des cheveux humides plus souples et plus lisses, ainsi que des cheveux séchés plus malléables et plus individualisés.

La présente invention a donc pour objet une composition de conditionnement des cheveux comprenant, dans un milieu aqueux cosmétiquement acceptable, au moins un tensioactif cationique choisi parmi les sels d'ammonium quaternaire contenant au moins une fonction ester et un mélange d'alcools gras tel que décrit ci-dessous.

Un autre objet de l'invention consiste en un procédé de traitement cosmétique des cheveux mettant en oeuvre la composition susvisée.

L'invention a encore pour objet une utilisation de ladite composition comme aprés-shampoing.

D'autres objets, caractéristiques, aspects et avantages de l'invention apparaîtront encore plus clairement à la lecture de la description et des divers exemples qui suivent.

Selon l'invention, la composition de conditionnement des cheveux comprend dans un milieu aqueux cosmétiquement acceptable, au moins un tensioactif cationique choisi parmi les sels d'ammonium quaternaire contenant au moins une fonction ester, un mélange d'alcools gras qui comprend an moins 60% en poids d'alcools gras en C₂₀₋₂₄ par rapport au poids total du mélange, et éventuellement des tensioactifs détergents en une quantité inférieure ou égale à 4 % en poids par rapport au poids total de la composition.

Le mélange d'alcools gras comprend au moins 60 % en poids, de préférence au moins 80 % en poids et plus particulièrement au moins 90 % en poids d'alcools gras en C₂₀₋₂₄, par rapport au poids total du mélange.

Les alcools grays en C₂₀₋₂₄ utilisables dans le mélange d'alcools de la présente invention comportent une chaîne hydrocarbonée linéaire ou ramifiée, saturée ou insaturée. De préférence, ce sont des alcools gras primaires qui présentent une chaîne linéaire et saturée. On peut notamment citer à titre d'exemples l'alcool béhénylique, l'alcool arachidylique, l'alcool lignocérylique et leurs mélanges.

La composition selon l'invention peut contenir également une quantité inférieure ou égale à 40 % en poids, de préférence inférieure ou égale à 20 % en poids, et plus particulièrement inférieure ou égale à 10 % en poids par rapport au poids total du mélange, d'au moins un alcool en C₁₄₋₁₉ tel que, par exemple, l'alcool cétylique et l'alcool stéarylique.

En outre, la composition selon l'invention peut contenir une quantité inférieure ou égale à 40 % en poids, de préférence inférieure ou égale à 20 % en poids, et plus particulièrement inférieure ou égale à 10 % en poids par rapport au poids total du mélange, d'au moins un alcool en C₂₅₋₄₀ tel que, par exemple, l'alcool cérylique et l'alcool montanylique.

Comme mélange d'alcools gras particulièrement préféré dans la composition selon l'invention, on peut utiliser, par exemple, le mélange d'alcools gras constitué de 76 % en poids d'alcool béhénylique, de 17 % en poids d'alcool arachidylique, de 1,5 % en poids d'alcool lignocérylique, de 5 % en poids d'alcool stéarylique et de 0,5 % en poids d'alcool cétylique. Ce mélange est vendu sous la dénomination Nafol® 1822 C par la société CONDEA. Comme autres exemples, on peut également citer le mélange vendu sous la dénomination Nafol® 2298 par la société CONDEA, qui comprend 98 % en poids d'alcool béhénylique ; le mélange vendu sous la dénomination Nafol® 20-22 par la société CONDEA, qui comprend 30 % en poids d'alcool béhénylique, 58 % en poids d'alcool arachidylique et 6 % en poids d'alcool lignocérylique ; ou encore le mélange vendu sous la dénomination Nafol® 20+ par la société CONDEA, qui comprend 50 % en poids d'alcool arachidylique, 29 % en poids d'alcool béhénylique, 14 % en poids d'alcool lignocérylique et 6 % en poids d'alcool stéarylique.

La quantité du mélange d'alcools gras dans la composition selon l'invention est notamment comprise entre 0,1 et 20 % en poids, de préférence entre 0,5 et 10 % en poids par rapport au poids total de la composition.

Les sels d'ammonium quaternaire contenant au moins une fonction ester, soit notamment ceux de formule (IV) suivante : 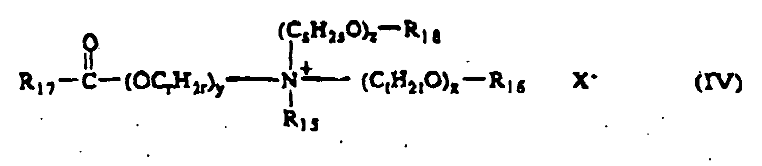 dans laquelle :
R₁₅ est choisi parmi les radicaux alkyles en C₁-C₆ et les radicaux hydroxyalkyles ou dihydroxyalkyles en C₁-C₄ :
R₁₄ est choisi parmi :
   - le radical

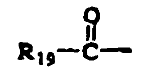

   - les radicaux R₂₀ hydrocarbonés en C₁-C₂₂, linéaires ou ramifiés, satures ou insaturés,
   - l'atome d'hydrogène,
      R₁₈ est choisi parmi :
   - la radical 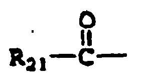
   - les radicaux R₂₁ hydrocarbonés en C₁-C₄, linéaires ou ramifiés, saturés ou insaturés.
   - l'atome d'hydrogène.
R₁₇, R₁₉ et R₂₁, identiques ou différents, sont choisis parmi les radicaux hydrocarbonés en C₇-C₂₁, linéaires ou ramifiés, saturés ou insaturés :
r, s et t, identiques ou différents, sont des entiers valant de 2 à 6 ;
y est un entier valant de 1 à 10 :
x et 2, identiques ou différents, sont des entiers valant de 0 à 10 ;
X' est un anion simple ou complexe, organique ou inorganique: sous réserve que la somme x + y + z vaut de 1 à 15, que lorsque x vaut 0 alors R₁₆ désigne R₂₀ et que lorsque z vaut 0 alors R₁₈, désigne R₂₂.

Les radicaux alkyles R₁₅ peuvent être linéaires ou ramifiés, et plus particulièrement linéaires.

De préférence, R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carboné.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1. Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1
- r, s et t sont égaux à 2 ;
- R₁₆ est choisi parmi :
   - le radical 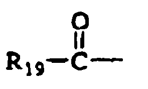
   - les radicaux méthyle, éthyle ou hydrocarbonés en C₁₄-C₂₂,
   - l'atome d'hydrogène ;
- R₁₈ est choisi parmi :
   - le radical 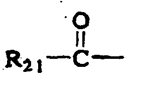
   - l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-mithylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société COGNIS, STEPANQUAT^{®} par la société STEPAN, NOXAMIUM^{®} par la société CECA, REWOQUAT^{®} WE 18 par la société REWO-GOLDSCHMIDT

La composition selon l'invention peut contenir de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-mithylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

A titre de sels d'ammonium quaternaire, on peut notamment citer, par exemple :
- ceux qui présentent la formule générale (I) suivante : 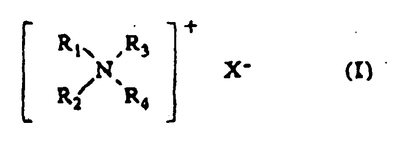 dans laquelle les symboles R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes; Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X' est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline comme, par exemple, ceux de formule (11) suivante : 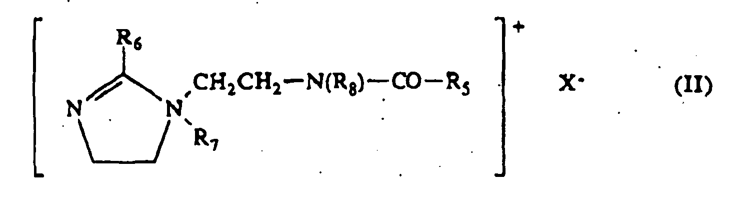 dans laquelle R₅ représente un radical alcényle ou alkyle comportant de 8 à 30 atomes de carbone, par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X' est un anion choisi dans le groupe des halogénures, phosphates, acétates, lactates, alkylsulfates, alkyl- ou alkylaryl-sulfonates. De préférence, R₅ et R₆ désignent un mélange de radicaux alcényle ou alkyle comportant de 12 à 21 atomes de carbone, par exemple derivés des acides gras du suif, R₇ désigne un radical méthyle, R₈ désigne un atome d'hydrogène. Un tel produit est par exemple commercialisé sous la dénomination REWOQUAT^{®} W 75 par la société REWO ;
- les sels de diammonium quaternaire de formule (III) : 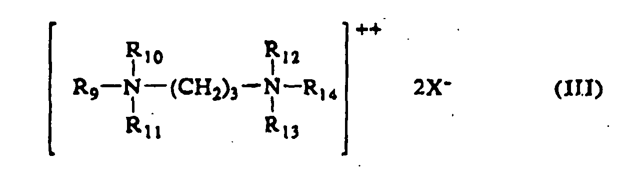 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X' est un anion choisi dans le groupe des halogénures, acétates, phosphates, nitrates et méthylsulfates. De tels sels de diammonium quaternaire comprennent notamment le dichlorure de propanesuif diammonium.

Parmi les sels d'ammonium quaternaire mentionnés ci-dessus, on préfère utiliser ceux répondant à la formule (I). On peut notamment citer d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acitate)-ammonium commercialisé sous la dénomination CERAPHYL^{®} 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium, le chlorure de cétyltrimethylammonium et le chlorure de palmitylamidopropyltritnéthylammonium.

La composition selon l'invention contient de préférence les tensioactifs cationiques en une quantité de 0,05 à 10 % en poids, de préférence de 0,1 à 5 % en poids par rapport au poids total de la composition.

La composition selon l'invention peut contenir éventuellement une quantité inférieure ou égale à 4 % en poids de tensioactifs détergents choisis parmi les tensioactifs anioniques, non ioniques, amphotères et leurs mélanges.

Les tensioactifs détergents utilisables dans la présente invention sont choisis parmi les tensioactifs anioniques, non ioniques et amphotères classiques bien connus dans la technique, et leurs mélanges.

La composition selon l'invention peut comprendre en outre au moins un agent conditionneur choisi parmi les silicones, les polymères cationiques, les esters, les huiles végétales, les huiles minérales, les huiles de synthèse telles que les poly(α-oléfines), et leurs mélanges.

Les silicones utilisables conformément à l'invention peuvent être solubles ou insolubles dans la composition, et elles peuvent être en particulier des polyorganosiloxanes insolubles dans la composition de l'invention. Elles peuvent se présenter sous forme d'huiles, de cires, de résines ou de gommes. Elles peuvent être utilisées pures ou en émulsion, en dispersion ou en microémulsion.

Les organopolysiloxanes sont définis plus en détail dans l'ouvrage de Walter NOLL "Chemistry and Technology of Silicones" (1968) Academic Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 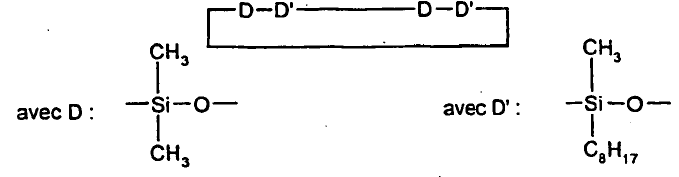 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,l'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Parmi les silicones non volatiles, on peut notamment citer les polyalkylsiloxanes, les polyarylsiloxanes, les polyalkylarylsiloxanes, les gommes et les résines de silicones, les polyorganosiloxanes modifiés par des groupements organofonctionnels ainsi que leurs mélanges.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732 ;
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255" ;
- des groupements hydroxyacylamino, comme les polyorganosiloxanes décrits dans la demande EP 342 834. On peut citer, par exemple, le produit Q2-8413 de la société DOW CORNING.

A titre d'exemples de silicones, on utilise de préférence les polydiméthylsiloxanes, les polyalkylarylsiloxanes, les polydiméthylsiloxanes à groupements aminés ou alcoxylés.

La composition selon l'invention peut comprendre en outre un ou plusieurs polymères cationiques. Par "polymère cationique", on entend tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont ceux décrits dans les brevets français n^{os} 2 505 348 et 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses; comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium ou de diméthyldiallylammonium ;
(4) les polysaccharides cationiques décrits plus particulièrement dans les brevets US 3 589 578 et 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2 162 025 et 2 280 361 ;
(6) les polyaminoamides solubles dans l'eau, tels que ceux notamment décrits dans les brevets français 2 252 840 et 2 368 508 ;
(7) les dérivés de polyaminoamides, par exemple, les polymères acide adipique/diakylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polylamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3 227 615 et 2 961 347 ;
(9) les cyclopolymères d'alkyldiallylamine ou de dialkyldiallylammonium tels que l'homopolymère de chlorure de diméthyldiallyl-ammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français 2 320 330, 2 270 846, 2 316 271, 2 336 434 et 2 413 907 et les brevets US 2 273 780, 2 375 853, 2 388 614, 2 454 547, 3 206.462, 2 261 002, 2 271 378, 3.874.870, 4.001.432, 3 929 990, 3 966 904, 4 005 193, 4 025 617, 4 025 627, 4 025 653, 4 026 945 et 4 027 020 ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F ;
(13) les polyamines comme le Polyquart® H vendu par HENKEL, référencées sous le nom de « POLYETHYLENEGLYCOL (15) TALLOW POLYAMINE » dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et
   leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on utilise de préférence les dérivés d'éther de cellulose quaternaires, les cyclopolymères cationiques, les polymères quaternaires de vinylpyrrolidone et de vinylimidazole, les polymères réticulés de sels de méthacryloyloxyalkyl(C₁-C₄) trialkyl(C₁-C₄)ammonium, les gommes de guar cationique et leurs mélanges.

La composition selon l'invention peut également comprendre un ou plusieurs esters d'acide gras, tels que par exemple, les composés de formule RₐCOOR_{b} dans laquelle Rₐ représente le reste d'un acide gras supérieur comportant de 4 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle, le néopentanoate d'isostéaryle et leurs mélanges.

La composition selon l'invention peut encore comprendre une ou plusieurs huiles végétales telles que l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum et leurs mélanges.

Comme huiles minérales, on peut notamment citer l'huile de paraffine et l'huile de vaseline.

Les agents conditionneurs, choisis parmi les silicones, les polymères cationiques, les esters, les huiles végétales, les huiles minérales, les huiles de synthèse et leurs mélanges, sont contenus de préférence dans la composition selon l'invention en une quantité allant de 0,01 % à 20 % en poids, mieux encore allant de 0,1 % à 10 % en poids et plus particulièrement allant de 0,5 % à 5 % en poids par rapport au poids total de la composition.

Le milieu aqueux cosmétiquement acceptable peut être constitué par de l'eau ou par un mélange d'eau et d'un solvant cosmétiquement acceptable tel qu'un alcool inférieur en C₁-C₄, par exemple l'éthanol, l'isopropanol, le tertio-butanol, le n-butanol ; les alkylèneglycols comme le propylèneglycol ; les éthers de polyols ; les alcanes en C₅-C₁₀ ; l'acétone, la méthyléthylcétone ; les acétates d'alkyle en C₁-C₄ comme l'acétate de méthyle, l'acétate d'éthyle, l'acétate de butyle ; le diméthoxyéthane, le diéthoxyéthane ; et leurs mélanges.

Le pH des compositions de l'invention est compris entre 3 et 8, de préférence entre 4 et 7.

Les compositions selon l'invention peuvent également contenir des additifs classiques bien connus dans la technique, tels que des polymères anioniques, non ioniques ou amphotères, des épaississants polymériques naturels ou synthétiques, anioniques, amphotères, zwittérioniques, non-ioniques ou cationiques, associatifs ou non, des épaississants non polymériques comme des acides ou des électrolytes, des opacifiants, des parfums, des colorants, des particules organiques ou minérales, des conservateurs, des agents de stabilisation du pH.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions cosmétiques selon l'invention peuvent se présenter sous forme de liquides fluides ou épaissis, de gels, de crèmes, de mousses, d'émulsions simples ou d'émulsions multiples.

Elles peuvent être utilisées, par exemple, comme après-shampoings, soins rincés, masques de soin profond, lotions ou crèmes de traitement du cuir chevelu.

Selon un mode de réalisation préféré de l'invention, la composition peut être utilisée comme après-shampoing, en particulier sur des cheveux fins.

La présente invention concerne également un procédé de traitement cosmétique des cheveux qui consiste à appliquer une quantité efficace d'une composition de conditionnement des cheveux telle que décrite ci-dessus, sur les cheveux qui sont notamment fins, à effectuer un éventuel rinçage après un éventuel temps de pose.

Les exemples suivants illustrent la présente invention et ne doivent être considérés en aucune manière comme limitant l'invention.

### EXEMPLES

Deux compositions d'après-shampoing sont préparées à partir des ingrédients indiqués dans le tableau suivant. La composition 1 est une composition selon l'invention tandis que la composition A est préparée à titre de comparaison.

| Composition | 1 | A |
|---|---|---|
| Chlorure de behentrimonium en solution à 80 % dans l'alcool isopropylique/eau (85/15) - GENAMIN KDMP de CLARIANT | 1,8 % | 1,8 % |
| PEG-180 | 2,0 % | 2,0 % |
| Mélange d'alcools Nafol® 1822C vendu par la société CONDEA | 3,0 % | - |
| Alcool cétylique | - | 3,0 % |
| Esters cétyliques | 0,25 % | 0,25 % |
| Amodiméthicone, Tridéceth-12, chlorure de cétrimonium (DC 939 de DOW CORNING) | 2,7 % | 2,7 % |
| Lanoline | 0,15 % | 0,15 % |
| Laurylméthicone-copolyol (DC 5200 de DOW) | 0,15 % | 0,15 % |
| Extrait végétal vendu sous la dénomination commerciale MFA Complex par BARNET | 0,1 % | 0,1 % |
| Hydroxyéthylcellulose | 0,2 % | 0,2 % |
| Pyrus malus | 0,5 % | 0,5 % |
| Vitamine B | 0,2 % | 0,2 % |
| Acide citrique | 0,02 % | 0,02 % |
| Parfum | qs | qs |
| Conservateurs | qs | qs |
| Eau qsp | 100 % | 100 % |

Les pourcentages indiqués dans le tableau ci-dessus sont des pourcentages en poids par rapport au poids total de la composition.

Les deux compositions ont chacune été appliquées sur 10 modèles pendant 1 à 5 minutes. On a ensuite rincé et séché les cheveux.

Les cheveux mouillés sont alors plus lisses et plus souples et les cheveux séchés sont plus malléables et plus individualisés avec la composition selon l'invention.

Par ailleurs, une analyse du dépôt d'alcools gras sur des mèches de cheveux naturels traités par 10 applications successives de la combinaison shampoing/après-shampoing (composition 1) par rapport à la combinaison shampoing/après-shampoing (composition A) a été faite.

On a ensuite extrait les alcools gras des cheveux avec un extracteur DIONEX ASE 200 en utilisant comme solvant le dichlorométhane dans les conditions suivantes :
- 2 cycles de désorption de 10 minutes à 70 °C,
- *flush* (terme technique anglais désignant la quantité de solvant à vidanger exprimée en pourcentage du volume de la cellule) de 60 %,
- pression de 70 bars.

La quantité d'alcools gras est mesurée par chromatographie en phase gazeuse et on obtient les résultats suivants :

| | Quantité d'alcools gras (mg/g de mèche) |
|---|---|
| Composition 1 | 1,5 |
| Composition A | 2,3 |

La composition selon l'invention conduit bien à un dépôt moins important d'alcools gras sur les cheveux. Les cheveux apparaissent nettement moins chargés avec la composition 1 par rapport à ceux traités avec la composition A.

## Revendications

1. Composition de conditionnement des cheveux, **caractérisée en ce qu'**elle comprend, dans un milieu aqueux cosmétiquement acceptable, au moins un tensioactif cationique choisi parmi les sels d'ammonium quaternaire contenant au moins une fonction ester, un mélange d'alcools qui comprend au moins 60% en poids d'alcools gras en C₂₀₋₂₄ par rapport au poids total du mélange et éventuellement des tensioactifs détergents en une quantité inférieure ou égale à 4 % en poids par rapport au poids total de la composition.

2. Composition selon la revendication 1, **caractérisée en ce que** le mélange d'alcools gras comprend au moins 80 % en poids, et plus particulièrement au moins 90 % en poids d'alcools gras en C₂₀₋₂₄ par rapport au poids total du mélange.

3. Composition selon la revendication 1 ou 2, **caractérisée en ce que** les alcools gras en C₂₀₋₂₄ sont choisis parmi l'alcool béhénylique, l'alcool arachidylique, l'alcool lignocérylique et leurs mélanges.

4. Composition selon l'une quelconque des revendications précédentes, **caractérisée en ce que** le mélange d'alcools gras comprend une quantité inférieure ou égale à 40 % en poids, de préférence inférieure ou égale à 20 % en poids, et plus particulièrement inférieure ou égale à 10 % en poids, par rapport au poids total du mélange, d'au moins un alcool en C₁₄₋₁₉.

5. Composition selon la revendication 4, **caractérisée en ce que** les alcools en C₁₄₋₁₉ sont choisis parmi l'alcool stéarylique, l'alcool cétylique et leurs mélanges.

6. Composition selon l'une quelconque des revendications précédentes, **caractérisée en ce que** le mélange d'alcools gras comprend une quantité inférieure ou égale à 40 % en poids, de préférence inférieure ou égale à 20 % en poids, et plus particulièrement inférieure ou égale à 10 % en poids, par rapport au poids total du mélange, d'au moins un alcool en C₂₅₋₄₀.

7. Composition selon l'une quelconque des revendications précédentes, **caractérisée en ce que** le mélange d'alcools gras est contenu en une quantité de 0,1 à 20 % en poids, de préférence de 0,5 à 10 % en poids par rapport au poids total de la composition.

8. Composition selon l'une quelconque des revendications précédentes, **caractérisée en ce que** les sels d'ammonium quaternaire contenant au moins une fonction ester sont choisis parmi ceux de formule (IV) suivante : 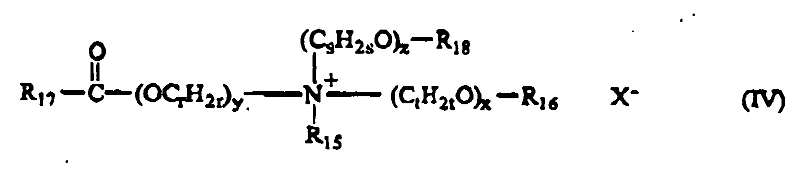 dans laquelle :
R₁₅ est choisi parmi les radicaux alkyles en C₁-C₆ et les radicaux hydroxyalkyles ou dihydroxyalkyles en C₁-C₆ ;
R₁₆ est choisi parmi : .
- le radical 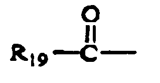
- les radicaux R₂₀ hydrocarbonés en C₁-C₂₂, linéaires ou ramifiés, saturés ou insaturés,
- l'atome d'hydrogène,
R₁₈ est choisi parmi :
- le radical 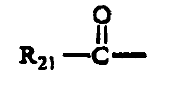
- les radicaux R₂₂ hydrocarbonés en C₁-C₆, alinéaires ou ramifiés, saturés ou insaturés,
- l'atome d'hydrogène,
R₁₇, R₁₉ et R₂₁, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X' est un anion simple ou complexe, organique ou inorganique ;
sous réserve que la somme x + y + z vaut de 1 à 15, que lorsque x vaut 0 alors R₁₆ désigne R₂₀ et que lorsque z vaut 0 alors R₁₈ désigne R₂₂.

9. Composition selon l'une quelconque des revendications précédentes, **caractérisée en ce que** les tensioactifs cationiques sont contenus en une quantité de 0,05 à 10 % en poids, de préférence de 0,1 à 5 % en poids par rapport au poids total de la composition.

10. Composition selon l'une quelconque des revendications précédentes, **caractérisée en ce que** les tensioactifs détergents sont choisis parmi les tensioactifs anioniques, non ioniques, amphotères et leurs mélanges.

11. Composition selon l'une quelconque des revendications précédente, **caractérisée en ce qu'**elle comprend en outre au moins un agent conditionneur.

12. Composition selon la revendication 11, **caractérisée en ce que** l'agent conditionneur est choisi parmi les silicones, les polymères cationiques, les esters, les huiles végétales, les huiles minérales, les huiles de synthèse et leurs mélanges.

13. Composition selon l'une quelconque des revendications précédentes, **caractérisée en ce que** le milieu aqueux cosmétiquement acceptable est constitué par de l'eau ou par un mélange d'eau et d'un solvant cosmétiquement acceptable.

14. Composition selon la revendication 13, **caractérisée en ce que** le solvant cosmétiquement acceptable est choisi parmi les alcools inférieur en C₁-C₄, les alkylèneglycols, les éthers de polyols, les alcanes en C₅-C₁₀, l'acétone, la méthyléthylcétone, les acétates d'alkyle en C₁-C₄, le diméthoxyéthane, le diéthoxyéthane, et leurs mélanges.

15. Composition selon l'une quelconque des revendications précédentes, **caractérisée en ce qu'**elle comprend en outre des additifs tels que des polymères anioniques, non ioniques ou amphotères, des épaississants polymériques naturels ou synthétiques, anioniques, amphotères, zwittérioniques, non-ioniques ou cationiques, associatifs ou non, des épaississants non polymériques comme des acides ou des électrolytes, des opacifiants, des parfums, des colorants, des particules organiques ou minérales, des conservateurs, des agents de stabilisation du pH.

16. Composition selon l'une quelconque des revendications précédentes, **caractérisée en ce qu'**elle est se présente sous la forme d'un après-shampoing pour cheveux fins.

17. Procédé de traitement cosmétique des cheveux, **caractérisé en ce que** l'on applique sur les cheveux, une quantité efficace de la composition de conditionnement des cheveux selon l'une quelconque des revendications précédentes, et on effectue un éventuel rinçage après un éventuel temps de pose.

18. Procédé de traitement cosmétique des cheveux selon la revendication 17, **caractérisé en ce que** l'on applique la composition sur des cheveux fins.

## Claims

1. Hair conditioning composition, **characterized in that** it comprises, in a cosmetically acceptable aqueous medium, at least one cationic surfactant chosen from quaternary ammonium salts containing at least one ester function, a fatty alcohol mixture that comprises at least 60% by weight of C₂₀₋₂₄ fatty alcohols relative to the total weight of the mixture, and optionally detergent surfactants in an amount of less than or equal to 4% by weight relative to the total weight of the composition.

2. Composition according to Claim 1, **characterized in that** the fatty alcohol mixture comprises at least 80% by weight and more particularly at least 90% by weight, of C₂₀₋₂₄ fatty alcohols relative to the total weight of the mixture.

3. Composition according to Claim 1 or 2, **characterized in that** the C₂₀₋₂₄ fatty alcohols are chosen from behenyl alcohol, arachidyl alcohol and lignoceryl alcohol, and mixtures thereof.

4. Composition according to any one of the preceding claims, **characterized in that** the fatty alcohol mixture comprises an amount of less than or equal to 40% by weight, preferably less than or equal to 20% by weight and more particularly less than or equal to 10% by weight, relative to the total weight of the mixture, of at least one C₁₄₋₁₉ alcohol.

5. Composition according to Claim 4, **characterized in that** the C₁₄₋₁₉ alcohols are chosen from stearyl alcohol and cetyl alcohol, and mixtures thereof.

6. Composition according to any one of the preceding claims, **characterized in that** the fatty alcohol mixture comprises an amount of less than or equal to 40% by weight, preferably less than or equal to 20% by weight and more particularly less than or equal to 10% by weight, relative to the total weight of the mixture, of at least one C₂₅₋₄₀ alcohol.

7. Composition according to any one of the preceding claims, **characterized in that** the fatty alcohol mixture is contained in an amount of from 0.1% to 20% by weight and preferably from 0.5% to 10% by weight relative to the total weight of the composition.

8. Composition according to any one of the preceding claims, **characterized in that** the quaternary ammonium salts containing at least one ester function are chosen from those of formula (IV) below: 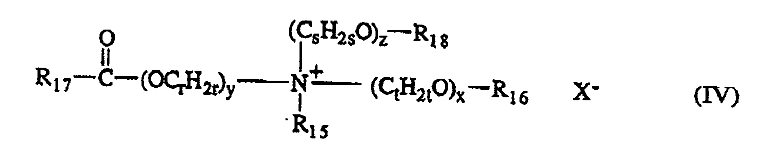 in which:
R₁₅ is chosen from C₁-C₆ alkyl radicals and C₁-C₆ hydroxyalkyl or dihydroxyalkyl radicals;
R₁₆ is chosen from:
- a radical 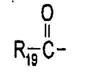
- linear or branched, saturated or unsaturated C₁-C₂₂ hydrocarbon-based radicals R₂₀,
- a hydrogen atom,
R₁₈ is chosen from:
- a radical 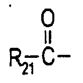
- linear or branched, saturated or unsaturated C₁-C₆ hydrocarbon-based radicals R₂₂,
- a hydrogen atom,
R₁₇, R₁₉ and R₂₁, which may be identical or different, are chosen from linear or branched, saturated or unsaturated C₇-C₂₁ hydrocarbon-based radicals;
r, s and t, which may be identical or different, are integers ranging from 2 to 6;
y is an integer ranging from 1 to 10;
x and z, which may be identical or different, are integers ranging from 0 to 10;
X⁻ is a simple or complex, organic or inorganic anion; with the proviso that the sum x + y + z is from 1 to 15, that when x is 0, then R₁₆ denotes R₂₀ and that when z is 0, then R₁₈ denotes R₂₂.

9. Composition according to any one of the preceding claims, **characterized in that** the cationic surfactants are contained in an amount of from 0.05% to 10% by weight and preferably from 0.1% to 5% by weight relative to the total weight of the composition.

10. Composition according to any one of the preceding claims, **characterized in that** the detergent surfactants are chosen from anionic, nonionic and amphoteric surfactants, and mixtures thereof.

11. Composition according to any one of the preceding claims, **characterized in that** it also comprises at least one conditioning agent.

12. Composition according to Claim 11, **characterized in that** the conditioning agent is chosen from silicones, cationic polymers, esters, plant oils, mineral oils and synthetic oils, and mixtures thereof.

13. Composition according to any one of the preceding claims, **characterized in that** the cosmetically acceptable aqueous medium consists of water or of a mixture of water and a cosmetically acceptable solvent.

14. Composition according to Claim 13, **characterized in that** the cosmetically acceptable solvent is chosen from C₁-C₄ lower alcohols, alkylene glycols, polyol ethers, C₅-C₁₀ alkanes, acetone, methyl ethyl ketone, C₁-C₄ alkyl acetates, dimethoxyethane and diethoxyethane, and mixtures thereof.

15. Composition according to any one of the preceding claims, **characterized in that** it also comprises additives such as anionic, nonionic or amphoteric polymers, natural or synthetic anionic, amphoteric, zwitterionic, nonionic or cationic polymeric thickeners, that may or may not be associative, non-polymer thickeners, for instance acids or electrolytes, opacifiers, fragrances, colorants, organic or mineral particles, preserving agents and pH stabilizers.

16. Composition according to any one of the preceding claims, **characterized in that** it is in the form of an after-shampoo conditioner for fine hair.

17. Cosmetic process for treating the hair, **characterized in that** an effective amount of the hair conditioning composition according to any one of the preceding claims is applied to the hair, and the composition is optionally rinsed out after optionally leaving it on the hair for a period of time.

18. Cosmetic process for treating the hair according to Claim 17, **characterized in that** the composition is applied to fine hair.

## Patentansprüche

1. Zusammensetzung zum Konditionieren der Haare, **dadurch gekennzeichnet, dass** sie in einem kosmetisch akzeptablen wässrigen Medium mindestens einen kationischen grenzflächenaktiven Stoff, der unter den quartären Ammoniumsalzen ausgewählt ist, die mindestens eine Esterfunktion besitzen, ein Gemisch von Alkoholen, das mindestens 60 Gew.-% C₂₀₋₂₄-Fettalkohole, bezogen auf das Gesamtgewicht des Gemisches, enthält, und gegebenenfalls reinigende grenzflächenaktive Stoffe in einer Menge von 4 Gew.-% oder darunter, bezogen auf das Gesamtgewicht der Zusammensetzung, enthält.

2. Zusammensetzung nach Anspruch 1, **dadurch gekennzeichnet, dass** das Gemisch von Fettalkoholen mindestens 80 Gew.-% C₂₀₋₂₄-Fettalkohole und insbesondere mindestens 90 Gew.-% C₂₀₋₂₄-Fettalkohole, bezogen auf das Gesamtgewicht des Gemisches, umfasst.

3. Zusammensetzung nach Anspruch 1 oder 2, **dadurch gekennzeichnet, dass** die C₂₀₋₂₄-Fettalkohole unter Behenylalkohol, Arachidylalkohol, Lignocerylalkohol und deren Gemischen ausgewählt sind.

4. Zusammensetzung nach einem der vorhergehenden Ansprüche, **dadurch gekennzeichnet, dass** das Gemisch von Fettalkoholen in einer Menge von 40 Gew.-% oder darunter, vorzugsweise höchstens 20 Gew.-% und insbesondere 10 Gew.-% oder darunter mindestens einen C₁₄₋₁₉-Fettalkohol, zogen auf das Gesamtgewicht des Gemisches, enthält.

5. Zusammensetzung nach Anspruch 4, **dadurch gekennzeichnet, dass** die C₁₄₋₁₉-Fettalkohole unter Stearylalkohol, Cetylalkohol und deren Gemischen ausgewählt sind.

6. Zusammensetzung nach einem der vorhergehenden Ansprüche, **dadurch gekennzeichnet, dass** das Gemisch von Fettalkoholen in einer Menge von 40 Gew.-% oder darunter, vorzugsweise höchstens 20 Gew.-% und insbesondere 10 Gew.-% oder darunter mindestens einen C₂₅₋₄₀-Fettalkohol, zogen auf das Gesamtgewicht des Gemisches, enthält.

7. Zusammensetzung nach einem der vorhergehenden Ansprüche, **dadurch gekennzeichnet, dass** das Gemisch von Fettalkoholen in einer Menge von 0,1 bis 20 Gew.-% und vorzugsweise 0,5 bis 10 Gew.-%, bezogen auf das Gesamtgewicht der Zusammensetzung, enthalten ist.

8. Zusammensetzung nach einem der vorhergehenden Ansprüche, **dadurch gekennzeichnet, dass** die quartären Ammoniumsalze, die mindestens eine Esterfunktion enthalten, unter den Verbindungen der folgenden Formel (IV) ausgewählt sind: 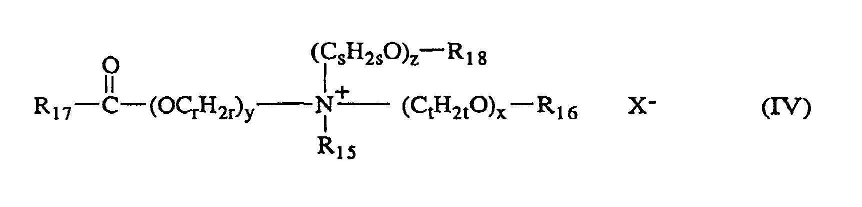 in der Formel:
R₁₅ ist unter den C₁₋₆-Alkylgruppen und den Hydroxyalkyl- oder Dihydroxyalkylgruppen mit 1 bis 6 Kohlenstoffatomen ausgewählt;
R₁₆ ist ausgewählt unter:
- der Gruppe 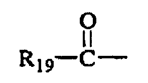
- den Gruppen R₂₀ auf Kohlenwasserstoffbasis mit 1 bis 22 Kohlenstoffatomen, die geradkettig oder verzweigt, gesättigt oder ungesättigt sind,
- dem Wasserstoffatom,
R₁₈ ist ausgewählt unter:
- der Gruppe 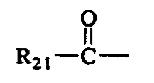
- den Gruppen R₂₂ auf Kohlenwasserstoffbasis mit 1 bis 6 Kohlenstoffatomen, die geradkettig oder verzweigt, gesättigt oder ungesättigt sind,
- dem Wasserstoffatom,
R₁₇, R₁₉ und R₂₁, die identisch oder voneinander verschieden sind, sind unter den geradkettigen oder verzweigten, gesättigten oder ungesättigten C₇₋₂₁-Kohlenwasserstoffgruppen ausgewählt; r, s und t, die gleich oder verschieden sind, sind ganze Zahlen von 2 bis 6;
y ist eine ganze Zahl von 1 bis 10;
x und z, die gleich oder verschieden sind, bedeuten 0 oder eine ganze Zahl von 1 bis 10;
X⁻ ist ein einfaches oder komplexes, organisches oder anorganisches Anion;
mit der Maßgabe, dass die Summe x + y + z im Bereich von 1 bis 15 liegt, dass R₁₆ R₂₀ bedeutet, wenn x 0 ist, und dass R₁₈ R₂₂ bedeutet, wenn z 0 ist.

9. Zusammensetzung nach einem der vorhergehenden Ansprüche, **dadurch gekennzeichnet, dass** die kationischen grenzflächenaktiven Stoffe in einer Menge von 0,05 bis 10 Gew.-% und vorzugsweise 0,1 bis 5 Gew.-%, bezogen auf das Gesamtgewicht der Zusammensetzung, enthalten sind.

10. Zusammensetzung nach einem der vorhergehenden Ansprüche, **dadurch gekennzeichnet, dass** die reinigenden grenzflächenaktiven Stoffe unter den anionischen, nichtionischen und amphoteren Tensiden und deren Gemischen ausgewählt sind.

11. Zusammensetzung nach einem der vorhergehenden Ansprüche, **dadurch gekennzeichnet, dass** sie ferner mindestens ein Konditioniermittel enthält.

12. Zusammensetzung nach Anspruch 11, **dadurch gekennzeichnet, dass** das Konditioniermittel unter den Siliconen, kationischen Polymeren, Estern, pflanzlichen Ölen, Mineralölen, synthetischen Ölen und deren Gemischen ausgewählt ist.

13. Zusammensetzung nach einem der vorhergehenden Ansprüche, **dadurch gekennzeichnet, dass** das kosmetisch akzeptable, wässrige Medium aus Wasser oder einem Gemisch von Wasser und einem kosmetisch akzeptablen Lösemittel besteht.

14. Zusammensetzung nach Anspruch 13, **dadurch gekennzeichnet, dass** das kosmetisch akzeptable Lösemittel unter den niederen C₁₋₄-Alkoholen, Alkylenglycolen, Polyolethern, C₅₋₁₀-Alkanen, Aceton, Methylethylketon, C₁₋₄-Alkylacetaten, Dimethoxyethan, Diethoxyethan und deren Gemischen ausgewählt ist.

15. Zusammensetzung nach einem der vorhergehenden Ansprüche, **dadurch gekennzeichnet, dass** sie ferner Zusatzstoffe enthält, wie anionische, nichtionische oder amphotere Polymere, natürliche oder synthetische, anionische, amphotere, zwitterionische, nichtionische oder kationische, assoziative oder nichtassoziative, polymere Verdickungsmittel, nichtpolymeren Verdickungsmittel, wie Säuren oder Elektrolyten, Trübungsmittel, Parfums, Farbmittel, organische oder anorganische Partikel, Konservierungsmittel und pH-Stabilisatoren.

16. Zusammensetzung nach einem der vorhergehenden Ansprüche, **dadurch gekennzeichnet, dass** sie in der Form eines Produktes für feine Haare vorliegt, das nach der Haarwäsche angewandt wird.

17. Verfahren für die kosmetische Behandlung der Haare, **dadurch gekennzeichnet, dass** eine Zusammensetzung zum Konditionieren der Haare nach einem der vorhergehenden Ansprüche in einer wirksamen Menge auf die Haare aufgebracht wird und nach einer gegebenenfalls abgewarteten Einwirkzeit gegebenenfalls gespült wird.

18. Verfahren für die kosmetische Behandlung der Haare nach Anspruch 17**, dadurch gekennzeichnet, dass** die Zusammensetzung auf feine Haare aufgetragen wird.
